# EUROPEAN PATENT APPLICATION

(11) **EP 1 304 080 A2**
(43) Date of publication of application: **23.04.2003**
(21) Application number: 02023403.5
(22) Date of filing: 18.10.2002
(51) Int. Cl.: A61B 17/16, A61B 17/82

(54) **Surgical ribbon file**

(30) Priority: 19.10.2001 JP 2001321360
(71) Applicant: Mani, Inc., Shioya-gun, Tochigi-ken 329-1234 (JP); Shiraishi, Tateru, Shioya-gun, Tochigi 329-1412 (JP)
(72) Inventor: Shiraishi, Tateru, Shioya-gun , Tochigi, 329-1412 (JP)
(74) Representative: Reinhard - Skuhra - Weise & Partner GbR

(57) **Abstract**

To provide a surgical ribbon file that can rapidly grind a projecting bone on a curved surface of a bone to an even face without skill and easily and safely grind a projecting bone in an opening between bones. The surgical ribbon file according to the present invention comprises a ribbon-shaped resilient main body with prescribed hardness and flexibility and file teeth on a surface of the main body. The ribbon-shaped main body may be made from alloy with high resiliency, synthetic resin or fiber-reinforced resin. Striking the ribbon-shaped main body from one face to the other face such that notches are formed and triangular portions project from the one face to the other face can form the file teeth, or the file teeth may be hemispherical projections standing on one face of the main body.

## Description

### BACKGROUND OF THE INVENTION

### 1.Field of the Invention

The present invention relates to a surgical ribbon file for grinding projections on bones, extraordinarily projecting portions or the like in orthopedic treatment and so on.

### 2. Description of the Related Art

For the above-mentioned purpose, a plate file, which has a lot of file teeth on the surface of hard tool steel, has been used in the same manner as an ordinary file for metal processing.

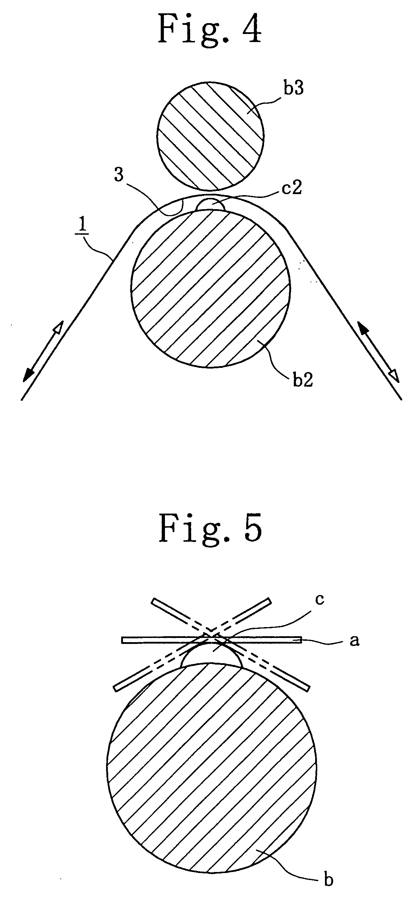

However, when the conventional plate file is used to smoothen a projecting bone (c) on a curved surface of a bone (b), operational technique and time are necessary to appropriately change the grinding angle of the plate file (a) as exemplified by the fancy lines in Fig. 5 while observing grinding condition. In addition, it is almost impossible to perform an expected grinding treatment when an extraordinarily projecting bone, which projects in a narrow opening between bones, should be ground with the conventional plate file. Forcibly performing the above operation may cause the surrounding tissue to be damaged by the plate file.

### SUMMARY OF THE INVENTION

The present invention has been made to solve the above problems and to provide a surgical ribbon file that can rapidly grind a projecting bone on a curved surface of a bone to an even face without skill and easily and safely grind a projecting bone in an opening between bones.

To accomplish the above objective, a surgical ribbon file according to the present invention comprises a ribbon-shaped resilient main body with prescribed hardness and flexibility and file teeth on a surface of the main body.

The resilient main body can be made from stainless steel, other steel, titanium-nickel alloy, titanium-aluminum alloy, other alloy with high resiliency, polyamide resin, polyether sulfone resin, polyethylene phthalate resin, other resins, carbon fiber, boron fiber, fiber-reinforced resins with other fibers, and so on. In these materials, steel, alloy with high resiliency or the like is suitable for ribbon files for normal grinding, and synthetic resin, fiber-reinforced resin or the like is suitable for ribbon files for finish grinding.

### BRIEF DESCRIPTION OF THE DRAWINGS

The present invention will be more apparent from the ensuring description with reference to the accompanying drawings wherein:
Figures 1A is a partially cutaway plan view of a ribbon file according to the present invention;
Figures 1B is a side view of a ribbon file according to the present invention;
Figure 1C is an enlarged cross-sectional view taken along the line X-X in Fig. 1A;
Figure 2A is a partially plan view of a ribbon file according to another embodiment of the present invention;
Figure 2B is a partially plan view of a ribbon file according to another embodiment of the present invention;
Figure 3 is a schematic view showing a usage of the surgical ribbon file according to the present invention;
Figure 4 is a schematic view showing another usage of the surgical ribbon file according to the present invention; and
Figure 5 is a schematic view showing a usage of a conventional plate file.

### DETAILED DESCRIPTION OF PREFERRED EMBODIMENTS

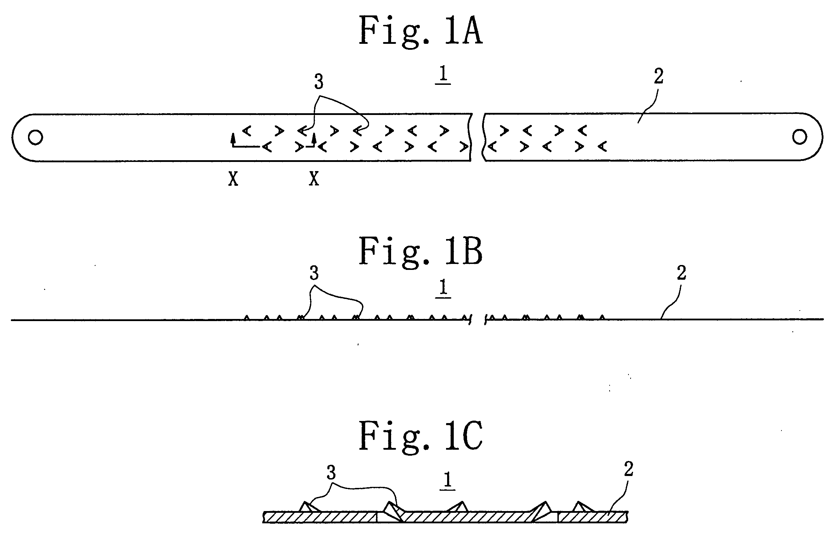

The ribbon file 1 shown in Fig. 1 according to a preferred embodiment of the present invention comprises a resilient ribbon main body 2, which is made from stainless steel (SUS301 or SUS304 regulated in Japanese Industrial Standard), and of which thickness is 0.1 mm, width is 5 mm and length is 300 mm, and a lot of file teeth 3 on one face of the main body 2.

In this embodiment, the above file teeth 3 are formed by pressing the main body 2 from one face to make a notch and projecting the tip of the triangular portion toward the other face as a sharp projection. Almost one half of the file teeth 3 are directed to a direction parallel to the longitudinal direction of the main body 2, and another half of the file teeth 3 are directed to a direction opposite to the direction of the above file teeth 3.

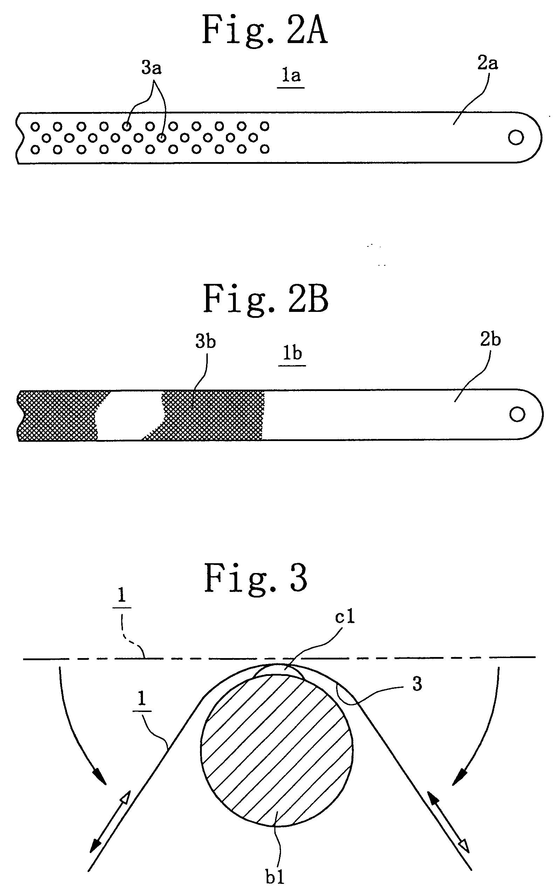

The ribbon file 1a illustrated in Fig. 2A comprises a resilient main body 2a, which is made from titanium-aluminum alloy with high resiliency, and of which thickness is 0.15 mm, width is 6 mm and length is 300 mm, and file teeth 3a as semispherical projections through pressing on one face of the main body 2a.

The ribbon file 1b illustrated in Fig. 2B comprises a resilient main body 2b, which is made from carbon-fiber-reinforced epoxy resin, and of which thickness is 0.2 mm, width is 7 mm and length is 300 mm, and file teeth 3b surrounded by laterally and longitudinally crossing channels with short distances like a lattice on one face of the main body 2b.

Working of the above ribbon files together with usage thereof will be explained below.

As illustrated in Fig. 3, when a projecting bone c1 of a bone b1 is smoothened to be flat, two end portions of the ribbon file 1 shown in Fig. 1 are grasped to put file teeth 3 in the middle of the ribbon file 1 on the projecting bone c1 as illustrated in the fancy line. Then, the ribbon file 1 is folded such that the both end portions close with each other, which causes the middle portion of the ribbon file 1 to bend almost along the curved surface of the bone b1. In this state, the both end portions of the ribbon file 1 are drawn one after another in a direction that the ribbon file 1 extends to grind the projecting bone c1 with the file teeth 3. In this grinding, the middle portion of the ribbon file 1 contacts the projecting bone c1 while the portion is always bent, so that only the continuous drawing operation of the both end portions of the ribbon file 1 forms the projecting bone c1 to be smoothly curved surface.

Next, when a projecting bone c2, which projects in a narrow opening between bones b2, b3 as shown in Fig. 4, is ground, the ribbon file 1 shown in Fig. 1 is inserted between the bones b2, b3, and the file teeth 3 in the middle of the ribbon file 1 is put on the projecting bone c2. Then, in the same manner as illustrated in Fig. 3, the ribbon file 1 is folded such that the both end portions close with each other, and the both end portions of the ribbon file 1 are drawn one after another. As a result, the projecting bone c2 is easily and smoothly ground without the surrounding tissue being damaged.

As described above, with the surgical ribbon file according to the present invention, in case that a projecting bone on a curved surface of a bone is ground, both end portions of the ribbon file is merely drawn in a direction that the file extends in a state that a portion of the ribbon file of the present invention is curved almost along the curved surface of the bone, which allows the projecting bone to be ground smoothly without skill. Further, in case that a projecting bone in an opening between bones is ground, the ribbon file of the present invention is inserted in the opening and the same operation as described above is performed, which allows the projecting bone to be ground easily without the surrounding tissue being damaged. As a result, the ribbon file according to the present invention will contribute to the improvement of surgical operations.

## Claims

1. A surgical ribbon file comprising:
a ribbon-shaped resilient main body with prescribed hardness and flexibility; and
file teeth on a surface of said main body.

2. The surgical ribbon file as claimed in claim 1,
wherein said ribbon-shaped main body is made from alloy with high resiliency.

3. The surgical ribbon file as claimed in claim 1,
wherein said ribbon-shaped main body is made from one of synthetic resin and fiber-reinforced resin.

4. The surgical ribbon file as claimed in claim 1,
wherein said file teeth are formed by striking said ribbon-shaped main body from one face to the other face such that notches are formed and triangular portions project from said one face to the other face.

5. The surgical ribbon file as claimed in claim 1,
said file teeth are hemispherical projections standing on one face of the main body.
